# EUROPEAN PATENT APPLICATION

(11) **EP 2 960 249 A1**
(43) Date of publication of application: **30.12.2015**
(21) Application number: 14173575.3
(22) Date of filing: 24.06.2014
(51) Int. Cl.: C07K 7/06, C12R 1/10, C07D 313/00

(54) **A bacillus licheniformis strain deposited under deposit number CECT 8307, isolated compounds and method for protecting or treating plants**

(71) Applicant: Symborg, S.L., 30100 Murcia (ES)
(72) Inventor: Juarez Molina, Jesus, 30100 Murcia (ES); Fernandez, Felix, 30100 Murcia (ES); Vicente Sanchez, Javier, 30100 Murcia (ES); Bernabe Garcia, Antonio J., 30100 Murcia (ES); Guillen Rubio, Juan, 30100 Murcia (ES); Torrecillas Sanchez, Alejandro, 30100 Espinardo, Murcia (ES); Gacto Fernandez, Marlano, 30100 Espinardo, Murcia (ES); Vicente Soler, Marla Jerónima, 30100 Espinardo, Murcia (ES)
(74) Representative: Carvajal y Urquijo, Isabel

(57) **Abstract**

A *Bacillus licheniformis* strain deposited under deposit number CECT 8307, comprising the sequence identified by SEQ ID NO: 1. A method for protecting or treating plants, plant parts, plant foliage, plant roots, plant seeds or fruits from insect infestations, comprising applying a composition comprising *Bacillus licheniformis* strain deposited under deposit number CECT 8307, comprising the sequence identified by SEQ ID NO: 1 or a supernatant obtained from a culture of *Bacillus licheniformis* strain deposited under deposit number CECT 8307, comprising the sequence identified by SEQ ID NO: 1 to said plant parts, plant foliage, plant roots, plant seeds or fruits. Compounds of formula (I), (II), (III), (IV) and (V) are also disclosed.

SEQUENCE LISTING

<110> SYMBORG, S. L.

<120> A Bacillus licheniformis STRAIN DEPOSITED UNDER DEPOSIT NUMBER CECT
8307, ISOLATED COMPOUNDS AND METHOD FOR PROTECTING OR TREATING PLANTS

<160> 1

<170> BiSSAP 1.2

<210> 1
<211> 1489
<212> DNA
<213> Bacillus licheniformis

<220>
<221> source
<222> 1..1489
<223> /mol_type="unassigned DNA"
      /note="Bacillus licheniformis CECT 8307 rRNA 16 S; complete
      sequence"
      /organism="Bacillus licheniformis"

<400> 1
atggctcagg acgaacgctg gcggcgtgcc taatacatgc aagtcgagcg gaccgacggg      60

agcttgctcc cttaggtcag cggcggacgg gtgagtaaca cgtgggtaac ctgcctgtaa     120

gactgggata actccgggaa accggggcta ataccggatg cttgattgaa ccgcatggtt     180

caatcataaa aggtggcttt cagctaccac ttgcagatgg acccgcggcg cattagctag     240

ttggtgaggt aacggctcac caaggcgacg atgcgtagcc gacctgagag ggtgatcggc     300

cacactggga ctgagacacg gcccagactc ctacgggagg cagcagtagg gaatcttccg     360

caatggacga aagtctgacg gagcaacgcc gcgtgagtga tgaaggtttt cggatcgtaa     420

aactctgttg ttagggaaga acaagtaccg ttcgaatagg gcggtacctt gacggtacct     480

aaccagaaag ccacggctaa ctacgtgcca gcagccgcgg taatacgtag gtggcaagcg     540

ttgtccggaa ttattgggcg taaagcgcgc gcaggcggtt tcttaagtct gatgtgaaag     600

cccccggctc aaccggggag ggtcattgga aactggggaa cttgagtgca gaagaggaga     660

gtggaattcc acgtgtagcg gtgaaatgcg tagagatgtg gaggaacacc agtggcgaag     720

gcgactctct ggtctgtaac tgacgctgag gcgcgaaagc gtggggagcg aacaggatta     780

gataccctgg tagtccacgc cgtaaacgat gagtgctaag tgttagaggg tttccgccct     840

ttagtgctgc agcaaacgca ttaagcactc cgcctgggga gtacggtcgc aagactgaaa     900

ctcaaaggaa ttgacggggg cccgcacaag cggtggagca tgtggtttaa ttcgaagcaa     960

cgcgaagaac cttaccaggt cttgacatcc tctgacaacc ctagagatag ggcttcccct    1020

tcgggggcag agtgacaggt ggtgcatggt tgtcgtcagc tcgtgtcgtg agatgttggg    1080

ttaagtcccg caacgagcgc aacccttgat cttagttgcc agcattcagt tgggcactct    1140

aaggtgactg ccggtgacaa accggaggaa ggtggggatg acgtcaaatc atcatgcccc 1200

ttatgacctg ggctacacac gtgctacaat gggcagaaca aagggcagcg aagccgcgag 1260

gctaagccaa tcccacaaat ctgttctcag ttcggatcgc agtctgcaac tcgactgcgt 1320

gaagctggaa tcgctagtaa tcgcggatca gcatgccgcg gtgaatacgt tcccgggcct 1380

tgtacacacc gcccgtcaca ccacgagagt ttgtaacacc cgaagtcggt gaggtaacct 1440

tttggagcca gccgccgaag gtgggacaga tgattggggt gaagtcgta 1489

## Description

### FIELD OF THE INVENTION

The present invention is in the field of biopesticides. More specifically, this invention relates to the finding that a novel strain of *Bacillus licheniformis,* CECT 8307, can suppress or control a range of difficult to control insect species, particularly those from the aphid family. The invention also relates to insecticidal compositions comprising this novel *Bacillus* strain and the metabolites, both alone and in combinations, produced by this strain, either alone, or in combination with other chemical and biological pesticides.

### BACKGROUND OF THE INVENTION

For some time, it has been known that certain microorganisms possess biological activity that is useful in the control of insect pests. While process has been made in identifying and developing biological pesticides, the majority of pesticides in use continue to be synthetic compounds. Many of these have undesirable side effects on non-target organisms, the environment, the users of these pesticides, as well as resulting in pesticide residues in food that many consumers would prefer to avoid.

Biological control offers an attractive alternative to synthetic chemical insecticides. Biopesticides can be safer to the environment and users, degrade more quickly, and will not result in chemical residues on treated crops. Biopesticides developed from microorganisms are extremely useful for integrated pest management programs and as tools to combat the development of pest resistance to synthetic chemicals. In addition, biopesticides can be developed in a shorter time period at less cost than synthetic pesticides.

Microbial natural products offer an abundant source of chemical diversity. Secondary metabolites produced by microbes (bacteria, actinomycetes, and fungi) provide novel chemical compounds which can be used either alone or in combination with known pesticides to effectively control insect pests.

Bacteria are the largest single group of microbial products, and the most commercially successful subset of the microbial biopesticides. The most widely used and longest used microbial biopesticides are subspecies and strains of *Bacillus thuringiensis* (Bt). Bt was first discovered in 1901 by Japanese biologist Shigetane Ishiwatari. Upon sporulation, Bt forms crystals of proteinaceous insecticidal δ-endotoxins, which bind to receptors in the insect's gut, causing cell lysis and death. Bt products have been in widespread commercial use as biological insecticides for more than 50 years. Commonly used subspecies include aizawai, israelensis, kurstaki, and tenebrionis with multiple strains available for each subspecies. Each strain of this bacterium produces a different mix of proteins, and specifically kills one or a few related species of insect larvae.

Other commonly used bacterial microbial biopesticides include *Bacillus subtilis* and *Bacillus amyloliquefaciens.* While primarily used to control fungal plant pathogens, *B*. *amyloliquefaciens* also has some activity against bacterial plant pathogens. *B*. *subtilis* bacteria produce a class of lipopeptide antibiotics which help *B*. *subtilis* bacteria out-compete other microorganisms by either killing them or reducing their growth rate. Multiple strains of *B*. *subtilis* are used commercially both in foliar and as seed treatment applications. *B*. *amyloliquefaciens* is very similar to *B*. *subtilis* in biological activity and use and *B*. *amyloliquefaciens* was previously classified by microbial taxonomists as a subspecies of *B*. *subtilis.*

*Bacillus licheniformis* is a Gram-positive endospore forming microorganism commonly found in the soil as well as marine sources which is also a member of the *B*. *subtilis* group (Caetano, et. al. Chemistry & Biology 18:90-100; Devi, P. et. al. Marine Drugs 8:1203-1212). The genome of B. licheniformis shows a high degree of similarity to B. subtilis, but contains defined regions that clearly are differentiated from B. subtilis at both the sequence and functional levels (Veith, B. et. al. J. Molecular Microbiology & Biotechnology 7:204-211). Previous agricultural investigations with *B*. *licheniformis* have focused on its use as a biocontrol agent of fungal pathogens (Tendulkar, et. al. J. of Applied Microbiology 103:2331-2339; US Patent Number 5,589,381 to Neyra; US Patent Number 5,665,354 to Neyra; US Patent Number 6,569,425 to Drahos; US Patent Number 6,824,772 to Drahos).

B. licheniformis has been shown to produce a number of secondary metabolites with potentially useful biocontrol properties. Lichenicidin is an antibacterial two component peptide antibiotic produced by *B*. *licheniformis* (Caetano, et. al. Chemistry & Biology 18:90-100). Surfactin, and the surfactin analogue lichenysin, have been isolated, purified, and characterized from *B*. *licheniformis* by multiple research groups (Compaoré, C. S. et. al. International Journal of Food Microbiology 162:297-307; Geetha, I. & Manonmani, A.M. " Letters in Applied Microbiology 41:406-412; Grangemard, I. et. al. Applied Biochemistry and Biotechnology 90:199-210; Konz, D. et. al. Journal of Bacteriology 181:133-140; Li, Y. et. al. Chemistry and Biodiversity 7:2065-2075; Pecci, Y. et. al. Journal of Mass Spectrometry 45:772-778; Tendulkar, et. al. J. of Applied Microbiology 103:2331-2339; Thaniyavarn, J. et. al. Biosci. Biotechnology, and Biochemistry 67:1239-1244; Yakimov, M.M. et. al. Biochimica et Biophysica Acta 1438:273-280). Other metabolites identified from B. licheniformis include novel bacteriocins (Compaoré, C. S. et. al. International Journal of Food Microbiology 162:297-307; He, L. et. al. Microbiological Research 161:321-326). Berka et. al. disclosed a number of polypeptides produced by *B*. *licheniformis* with cellulase, endoglucanase, or butyrate-acetoacetate CoA transferase activity (US Patent Number 7,494,798 to Berka; US Patent Number 7,863,032 to Berka; US Patent Number 8,168,417 to Berka). Last, thermostable chitinase enzymes have also been isolated from B. licheniformis strains (Khiyami M. and Masmali, I. Australian Journal of Basic & Applied Sciences 2:943-948).

As mentioned previously, most studies with B. licheniformis have focused on potential use as a biofungicide. Several studies have suggested potential bioinsecticidal uses. Ateyyat et al. found B. licheniformis among 11 whitefly associated bacteria. Although several other bacterial species showed greater potential, B. licheniformis did demonstrate some toxicity to whitefly instars (Jordan Journal of Biological Sciences 2:139-144). Isolation of thermostable chitinases produced by B. licheniformis in red palm weevil larvae also suggests potential bioinsecticidal properties (Khiyami M. and Masmali, I. Australian Journal of Basic & Applied Sciences 2:943-948).

Aphids are among the most widespread plant pests which feed on the sap of plants using piercing sucking mouth parts. Over 250 species of aphids are serious plant pests, with some feeding exclusively on one plant species while others *(Myzus persicae)* feed on hundreds of plant species. Thrips species feed on a wide variety of plants using piercing sucking mouth parts. Over 5000 species of thrips have been identified with the number of species feeding on plants numbering in the 100's. Thrips also act as vectors for plant diseases and over 20 plant viruses are known to be transmitted by thrips. Whiteflies are tiny, sap-sucking insects that are frequently abundant in vegetable and ornamental plantings. Most whiteflies, especially the most common pest species-greenhouse whitefly *(Trialeurodes vaporariorum*) and silverleaf or sweetpotato whiteflies *(Bemisia* species)-have a wide host range that includes many weeds and crops. Plants infested with aphids, thrips, and whiteflies can have a variety of symptoms, such as decreased growth rates, mottled leaves, yellowing, stunted growth, curled leaves, browning, wilting, low yields and death. Aphids, thrips, and whiteflies also frequently transmit plant diseases, particularly viruses.

In the absence of control, aphids, thrips, and whiteflies can cause serious losses, both from the direct impact of feeding and through transmission of plant diseases. Chemical pesticides with systemic properties can provide control, but recent concerns with adverse non-target effects on bees related to neonicotinoid insecticides has removed one of the most successful chemical control options. In addition, aphids, thrips, and whiteflies can develop pesticide resistance quickly due to short life cycles if chemical insecticides with similar modes of action are used repeatedly. Biological controls with predators, such as lady beetles, green lacewings, or parasitic wasps, can provide control in greenhouses and other protected culture environments, but are not highly effective in field crops due to the high numbers of predators required.

### DESCRIPTION OF THE INVENTION

The present invention provides a *Bacillus licheniformis* strain deposited under deposit number CECT 8307, comprising the sequence identified by SEQ ID NO: 1, in the following, strain of the invention.

The strain of *Bacillus licheniformis* of the invention was deposited on 21 March, 2013 on the Colección Española de Cultivos Tipo (CECT), Parc Científic Universitat de Valencia, Catedrático Agustín Escardino, 9, 46980 Paterna (Valencia, Spain) by the depositor SYMBORG SL, sited at Campus de Espinardo 7. Edificio CEEIM, 30100 Murcia (Spain).

The strain of the invention was identified by the depositor with the reference SB0016, and received the CECT accession number CECT 8307 after the International Depositary Authority declared the strain as viable.

The strain of the invention was identified based on the utilization of molecular characterization, physiological and proteomic study. Microscopic observation identified the strain is formed by Gram-positive bacilli, capable of sporulation, therefore belonging to the genus Bacillus. The sample was seeded using various liquid and solid media, where growth was found after incubation at between 30 and 50 °C. Isolated colonies of the strain were then subjected to biochemical tests, the results of which are listed in the table below.

A more extensively characterization of the strain of the invention was conducted by Colección Española de Cultivos Tipo (CECT).

Isolate Identification: Using API strips, specifically API 50CHE, following the manufacturer's instructions (Biomerieux), which advises use of the API 20E gallery to complement the API 50CH gallery, and an incubation at 37 °C. Readings were conducted following 24 and 48 hours incubation at the optimal growth temperature of the strain under aerobic conditions.

Cellular morphology: The motile cells are found singly, with two endospore formed on the subterminal region. The cells are uniformly stained Gram positive.

Colony morphology: The colonies are opaque and entire with low convex elevation, glistening and smooth appearance. The margins are white and regular.

**Table 1. API 50CHE gallery Results**

| | | | |
|---|---|---|---|
| Rods | + | Colony entire | + |
| Rods straight | + | Colony erose | + |
| Rods curved | - | Colony lobate | - |
| Cell singles | + | Colony circular | - |
| Ends tarpered | - | Colony irregular | + |
| Ends rounded | + | Colony rhizoid | + |
| Ends squared | - | Colony low convex | + |
| Endospore formed | + | Colony high convex | - |
| Sporanggium swollen | - | Colony flat | - |
| One spore/cell | - | Colony raised | - |
| Spore round | - | Colony glistening | + |
| Spore cylindrical | + | Colony dull | - |
| Spore oval | - | Colony dry | - |
| Spore central | - | Colony smooth | - |
| Spore terminal | + | Colony rough | + |
| Spore subterminal | - | Catalase | + |
| Gram positive | + | Insoluble Brown pigment | - |
| Gram negative | - | Insoluble Black pigment | - |
| Gram variable | - | Insoluble yellow pigment | - |
| Gram stained | + | Insoluble orange pigment | - |
| Vacuoles present | + | Insoluble red pigment | - |
| Colony translucent | - | Soluble Brown pigment | - |
| Colony Transparent | - | Soluble Black pigment | - |
| Cells motile | + | Soluble yellow pigment | - |
| Growth at 15° C | + | Oxidase | + |
| Growth at 26° C | + | Aerobe | + |
| Growth at 30° C | + | Facultative | - |
| Growth at 37° C | + | Microaerophile | + |
| Growth at 45° C | + | Anaerobe | + |
| Growth at 50° C | + | Growth at pH 4.5 | + |
| Growth at 55° C | + | Growth at pH 6.5 | + |
| Growth at 55° C | - | Growth at pH 9 | + |
| Growth at 60° C | - | Growth at 2 % NaCl | + |
| Starch Hidrolysis | + | Growth at 5 % NaCl | + |
| Colony opaque | + | Growth at 10 % NaCl | + |

**Table 2. API 20E gallery Results**

| **Tube** | **Substrate** | **Enzyme** | **24 h** | **48h** |
|---|---|---|---|---|
| ONPG | 2-nitro-fenil-βD-galactopiranoside | β-galactosidase | + | + |
| ADH | L-arginine | Arginine dihidrolase | - | - |
| LDC | L-lysine | Lysine descarboxilase | - | - |
| ODC | L-ornithine | Ornithine descarboxilase | - | - |
| CIT | Trisodium Citrate | Citrate utilization | + | + |
| H2S | Sodium thiosulfate | H₂S Production | - | - |
| URE | Urea | Urease | - | - |
| TDA | L- Tryptophane | Tryptophane desaminase | - | - |
| IND | L- Tryptophane | Indole Production | - | - |
| VP | Piruvate sódico | Acetoine Production | + | + |
| GEL | Gelatin bovina | Gelatinase | + | + |
| GLU | D-Glucose | Oxidation/Fermentation | - | - |

Additional analysis of proteins was determined following the protocol recommended by Bruker Daltroniks by the extraction of ethanol/formic acid. Spectrograms showed a close correlation between this strain and known *B*. *licheniformis* strains.

Finally, genetic sequence of *Bacillus licheniformis* strain CECT 8307 was completed, and found to match to greater than 99.8% similarity, with more than 60 different *B*. *subtilis* strains. Based upon this analysis, it was concluded that strain CECT 8307 is a member of *Bacillus licheniformis.* In addition, the peptide fingerprint of strain CECT 8307 through 2D electrophoresis differs from other species with regard to the protein content present in the NCBI databank.

Based on all available physiological and biochemical data, the strain CECT 8307 most closely resembles *Bacillus licheniformis.*

The present invention also provides a a method for protecting or treating plants, plant parts, plant foliage, plant roots, plant seeds or fruits from insect infestations, comprising applying
- a composition comprising *Bacillus licheniformis* strain deposited under deposit number CECT 8307, comprising the sequence identified by SEQ ID NO: 1 or
- a supernatant obtained from a culture of *Bacillus licheniformis* strain deposited under deposit number CECT 8307, comprising the sequence identified by SEQ ID NO: 1 to said plant parts, plant foliage, plant roots, plant seeds or fruits, in the following, method of the invention.

Another embodiment is the method of the invention, wherein said insect infestations are caused by foliar insects which attack the plant foliage or fruits.

Another embodiment is the method of the invention, wherein said insect infestations are caused by soil insects which attack plant roots.

Another embodiment is the method of the invention, wherein said insect infestations are caused by an insect selected from the group consisting of aphids, whiteflies and thrips.

Another embodiment is the method of the invention, wherein said insect infestations are caused by a member of a family selected from the group consisting of Aphididae, Aleyrodidae and Thripidae.

Another embodiment is the method of the invention, wherein said insect infestations are caused by an insect selected from the group consisting of *Myzus* spp., *Aphis* spp., *Bemisia* spp., *Trialeurodes* spp., *Frankliniella* spp. and *Thrips* spp.

Another embodiment is the method of the invention, wherein a biological or chemical pesticide is further applied to said plant parts, plant foliage, plant roots, plant seeds or fruits.

Another embodiment is the method of the invention, wherein *Bacillus licheniformis* strain deposited under deposit number CECT 8307 is applied as a whole broth culture.

Another embodiment is the method of the invention, wherein *Bacillus licheniformis* strain deposited under deposit number CECT 8307 is applied to the soil surrounding the plant roots.

Another embodiment is the method of the invention, wherein *Bacillus licheniformis* strain deposited under deposit number CECT 8307 is applied as wettable powders, granules, aqueous suspensions, emulsifiable concentrates or microencapsulations.

Another embodiment is a compound of formula (I)

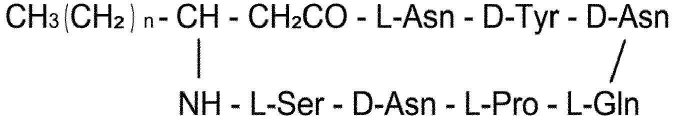

Another embodiment is a compound of formula (II) 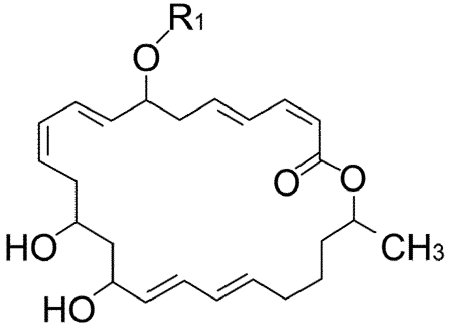 wherein R₁ is selected from the group consisting of H, CO-CH₂-COOH, CO-CH₂-CH₂-COOH and

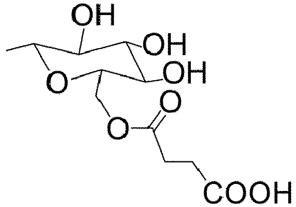

Another embodiment is a compound of formula (III) 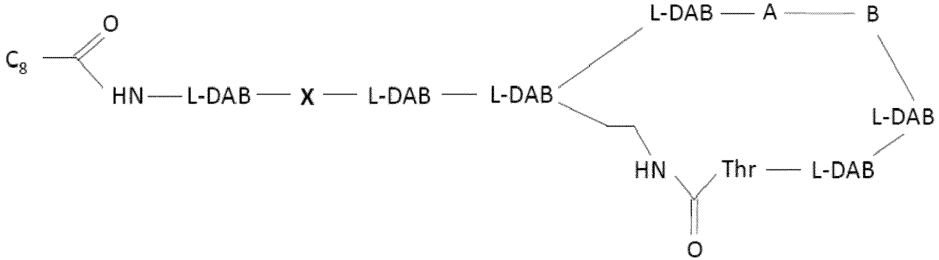 wherein X is selected from the group consisting of Met, Thr-Ala, Asn and Thr, A is selected from D-Leu and D-Phe, B is selected from the group consisting of Thr, Leu, Phe and ILeu, C₈ is 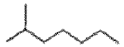 or 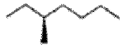 , L-DAB is 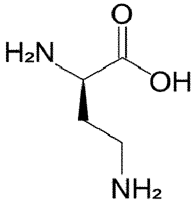 , provided that if X is Thr, B is Phe.

Another embodiment is a compound of formula (IV) 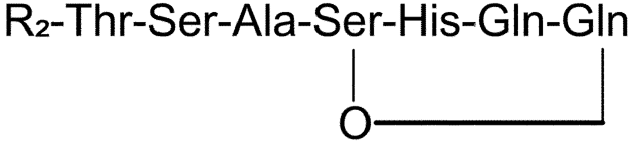 wherein R₂ is CH₃-CH₂-CH₂-CH₂-CH₂-CH₂-CH₂-CH₂-CH₂-CH₂-CH₂-

Another embodiment is a compound of formula (V)

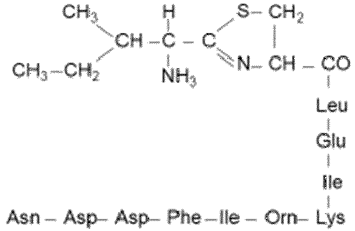

Another embodiment is a method for protecting or treating plants, plant parts, plant foliage, plant roots, plant seeds or fruits from insect infestations, comprising applying any of the compounds cited above to said plant parts, plant foliage, plant roots, plant seeds or fruits.

### BRIEF DESCRIPTION OF THE FIGURES

Figures 1-5 *Myzus nicotianae* percent mortality following treatment with *Bacillus licheniformis* CECT 8307 fractions.

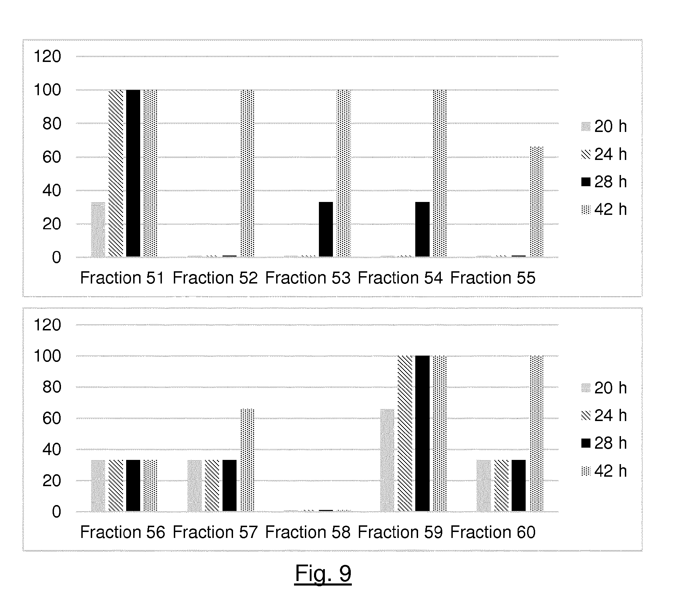

Figures 6-9. *Aphis gossypii* percent mortality following treatment with *Bacillus licheniformis* CECT 8307 fractions.

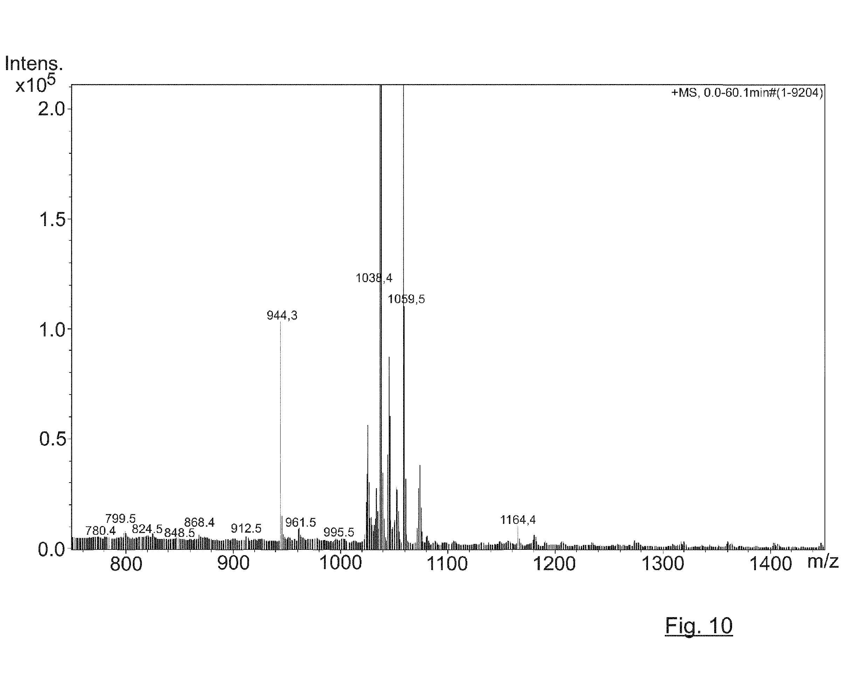

Figure 10. Mass spectra of *Bacillus licheniformis* strain CECT 8307 metabolites (700 - 1500 m/z)

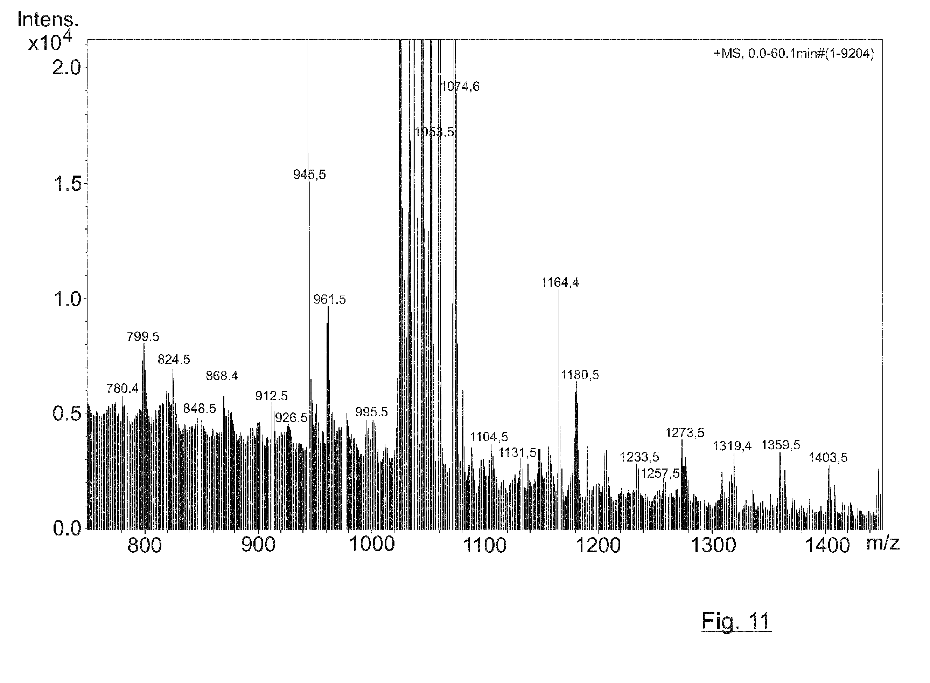

Figure 11. Mass spectra of *Bacillus licheniformis* strain CECT 8307 metabolites (700 - 1500 m/z) detail amplified.

### EXAMPLES OF THE INVENTION

### Example 1. Confirmation of toxicity of Bacillus licheniformis CECT 8307 against Myzus nicotianae

A nutrient agar rich in sucrose was distributed in each well of a 96-well microplate. Four to five adult aphids were placed in each well. To each well, a fraction of *Bacillus licheniformis* CECT 8307 (fractions numbered from 1 to 60 based on one minute collection times) dissolved in a 3:1 water:methanol mixture. Control treatments included 100% water, 100% methanol, and the 3:1 water:methanol mixture. The microplate wells were covered with an oxygen interchange membrane and evaluated for percentage mortality at 20, 24, 28, and 42 hours after treatment.

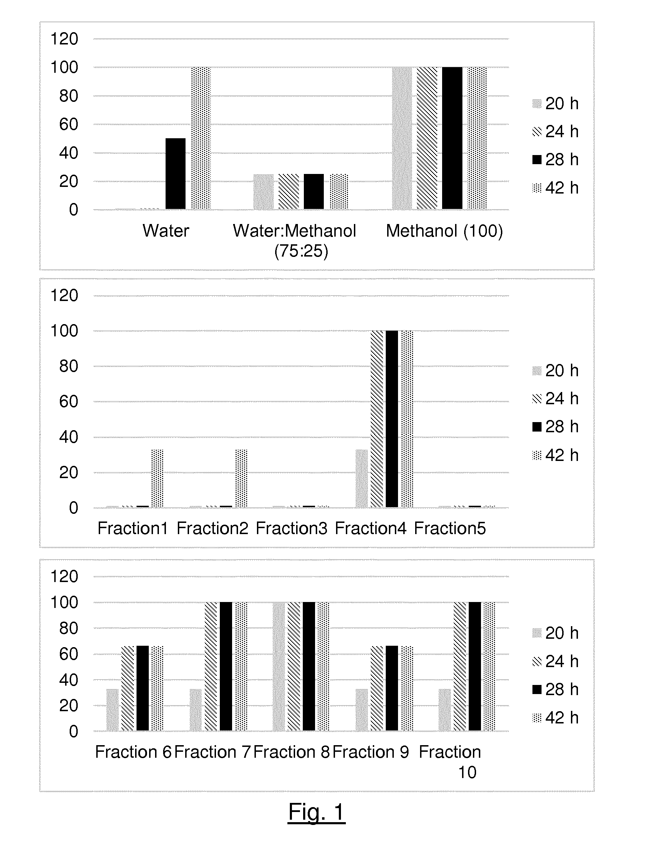

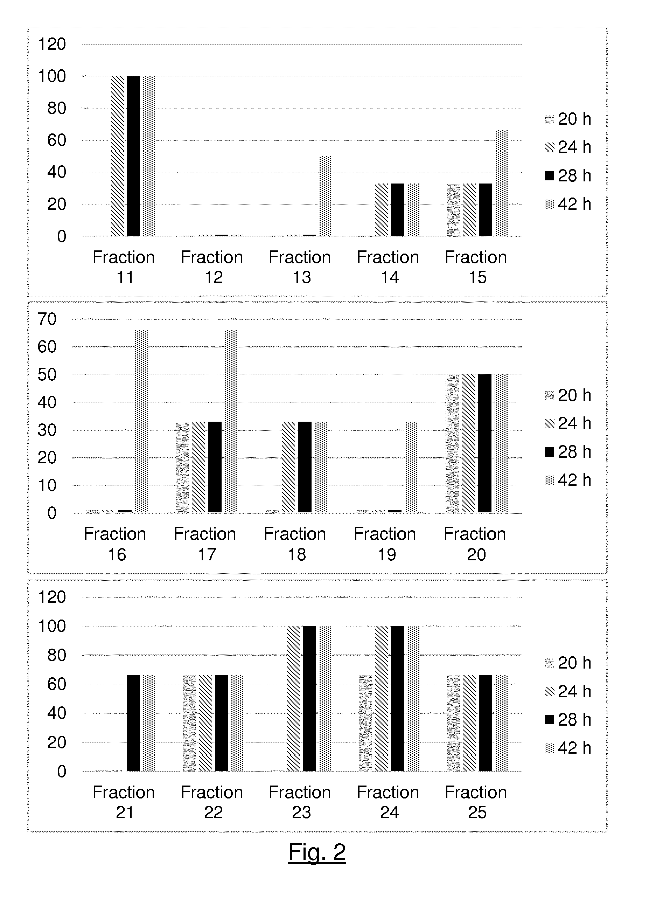

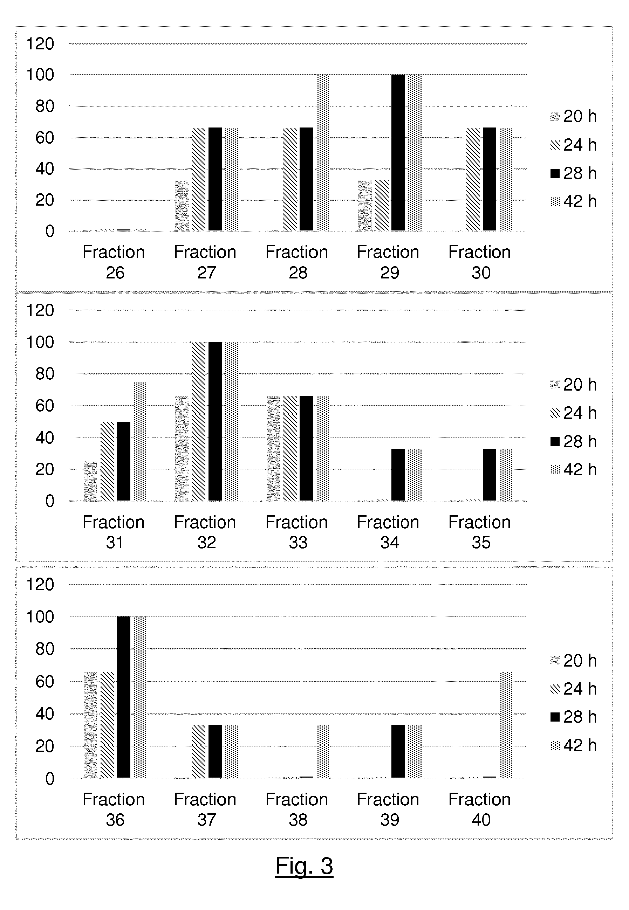

Results of testing are shown in Figures 1-3. Interesting activity was observed in fractions 4, 7, 8, 10, 11, 23, 24, 32, 36, 41, 44, 45, 48, 54, 55, 57, 59, and 60. The 3:1 water:methanol mixture showed consistent low level of mortality at each evaluation time.

### Example 2. Confirmation of toxicity of Bacillus licheniformis CECT 8307 against Aphis gossypii

A nutrient agar rich in sucrose was distributed in each well of a 96-well microplate. Four to five adult aphids were placed in each well. To each well, a fraction of *Bacillus licheniformis* CECT 8307 (fractions numbered from 1 to 60 based on one minute collection times) dissolved in a 1:1 water: methanol mixture. Control treatments included 100% water, 100% methanol, and the 1:1 water: methanol mixture. The microplate wells were covered with an oxygen interchange membrane and evaluated for percentage mortality at 20, 24, 28, and 42 hours after treatment.

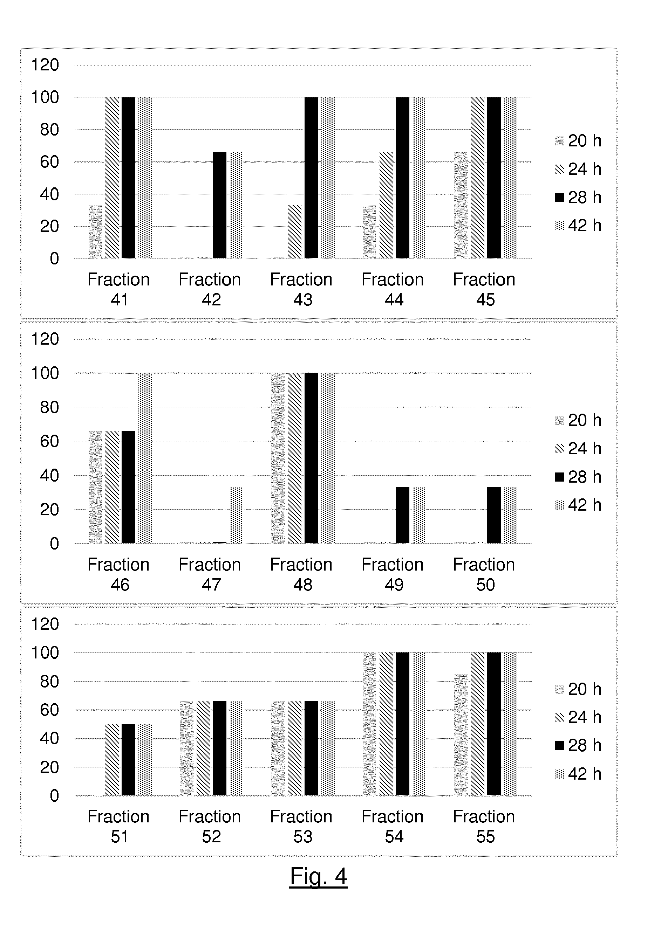

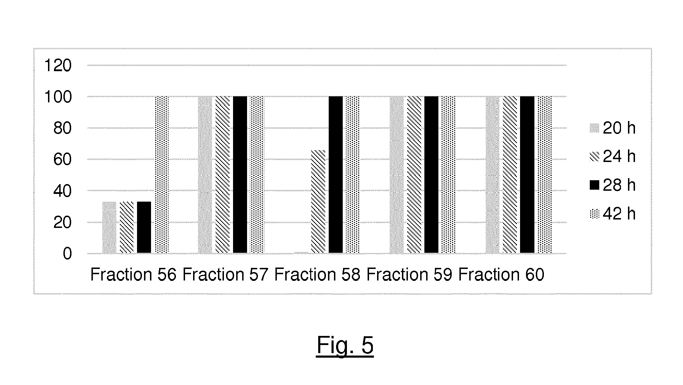

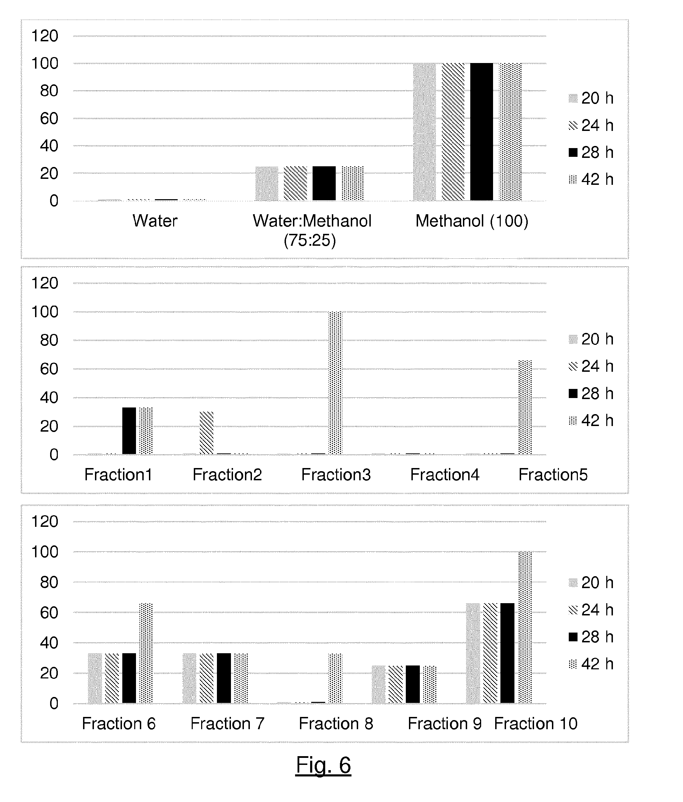

Results of testing are shown in Figures 4-6. Interesting activity was observed in fractions 13, 14, 15, 16, 18, 24, 26, 28, 42, 43, 54, 55, and 60. The 1:1 water: methanol mixture showed very low level of mortality at the first three evaluation times and increased to 50% mortality at the final time point.

### Example 3. Separation, Analysis and Activity of the Compounds Present in CECT 8307

Initially, the complete lipopeptides fraction were extracted from the bacterial supernatant and its mass spectra were obtained using an Agilent TOF 6000 Series Mass Spectrometer (Agilent Technologies, Santa Clara, CA, USA), equipped with an AP-MALDI Ion Source with a N2 laser (337 nm). Mass spectra were acquired by accumulating 200 laser shots in reflectron mode in positive mode in the range from 100-2200 m/z. The main TOF-MS parameters were as follows: capillary temperature, 350 °C; capillary voltage, 3500 V; fragmentor voltage, 215 V; skimmer voltage, 60 V; octopole DC1 and DC2 voltage, 34.4 and 34.2 V; octopole RF, 250 V.

Once the optimum culture was selected, the separation, fraction collection, and analysis of compounds present in the selected bacterial culture supernatant was performed with a HPLC/MS system. The bacterial supernatant was injected onto a C18 HPLC column. After injection, the column was washed with water/acetonitrile/formic acid for five minutes. Then compounds were separated using a linear gradient from 0% to 100% water/acetonitrile/formic acid for 30 minutes. The column was coupled to a fraction collector and 60 fractions (1 minute each) were collected and used for biological activity screening.

Biological activity of the collected lipopeptide fractions was screened against *Myzus nicotianae* and *Aphis gossypii* (see examples 1 and 2 for details). While biological activity against individual species was observed in a number of fractions, multiple fractions demonstrated activity against both insect species. The lipopeptides present and excreted by CECT 8307 in the active fractions previously separated by HPLC, are listed in Tables 3 and 4.

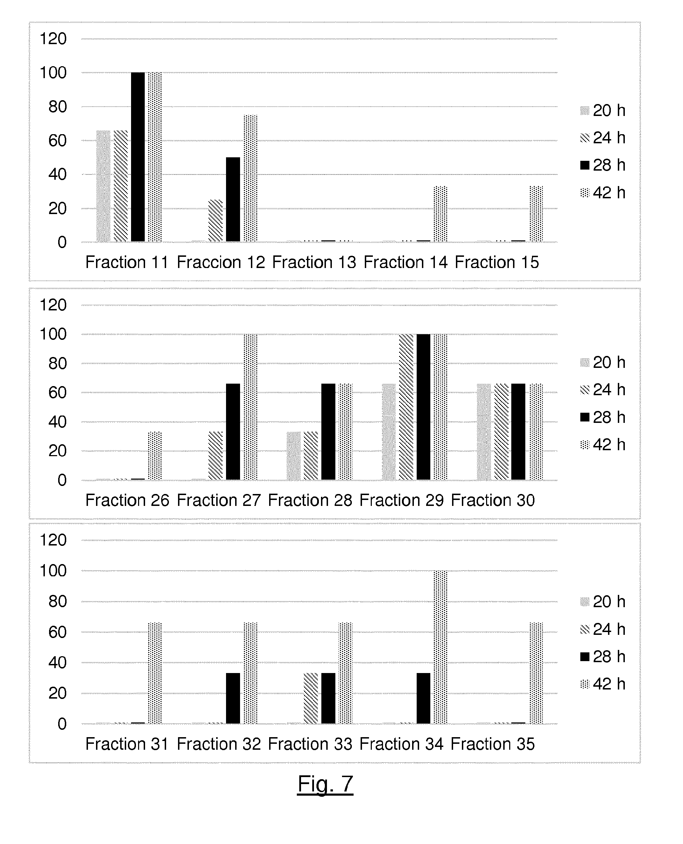

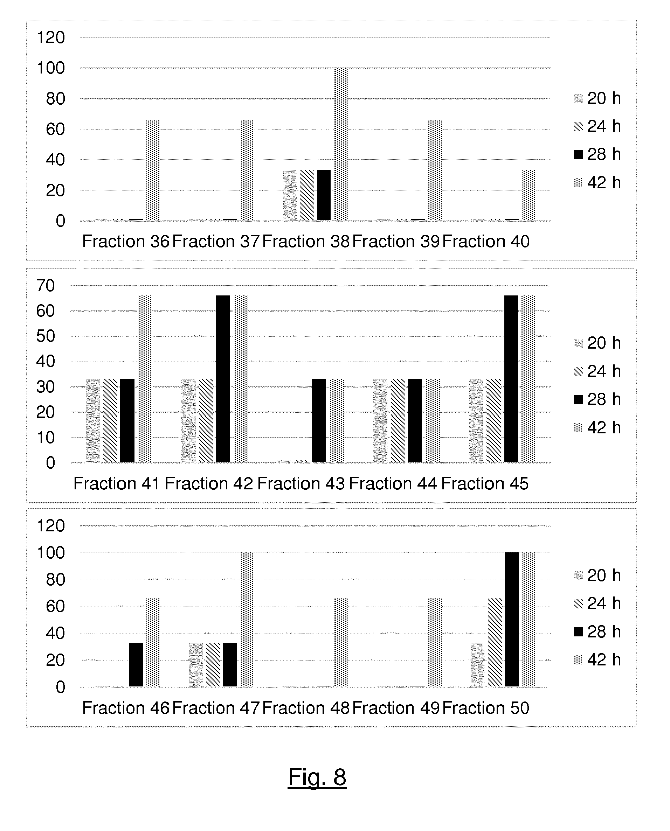

A combined MALDI-TOF mass spectrometry, LC/Mass and amino acids analysis of these fractions were used to identify a number of lipopeptide components, including surfactin, iturins, mycosubtilin, polymyxin, bacillomycin, putative kurstakins, and fengycin. Among these was an iturin, belonging to the iturin A family, not previously described in patents or the literature, found in fractions 24, macrolactins found in fraction 28, two kurstakins with new amino acid substitutions in fraction 28, several new polymyxin B1 with novel amino acid substitutions in fraction 32, a new bacitracin in fraction 32 with novel amino acid substitution, and a new polymyxin D2 with novel amino acid substitutions. The new characterized metabolites are highlighted in Tables 3-5. Figures 7 and 8 show the spectra of active metabolites produced by CECT 8307 from 700 to 1500 m/z.

**Table 3. Lipopeptides present and excreted by Bacillus licheniformis strain CECT 8307 in the active fractions against Aphis gosiipy previously separated by HPLC.**

| m/z | Intensity | Compound name |
|---|---|---|
| Fraction min 18 | | |
| 988.4 | 21657 | Iturina |
| 1137.4 | 5367 | Cyanidin 3-(6-malonylglucoside)-7-(6-feruloylglucoside)-3'-glucoside |

| Fraction min 50 | | |
|---|---|---|
| 669.4 | 8172 | Myrtucommulone A |
| 924.5 | 7188 | Amphotericin B |
| 1024.5 | 479236 | Iturin C10 + K |
| 1032.5 | 63464 | Surf C13 + K |
| 1036.6 | 14390059 | Surf C15 |
| 1043.8 | 776713 | Surf C14 + Na |
| 1050 | 84347 | Surf C16 |
| 1058.3 | 6029883 | Surf C15 + Na |

| Fraction min 51 | | |
|---|---|---|
| 668.4 | 6482 | Peonidin 3-glucoside-5-(6"-acetylglucoside) |
| 733.2 | 12137 | Kurilensoside J |
| 1024.4 | 432089 | Iturin C10 + K |
| 1036.4 | 19003010 | Surf C15 |
| 1043.7 | 522047 | Surf C14 + Na |
| 1058.2 | 6701109 | Surf C15 + Na |
| 1073.7 | 211604 | Iturin C16 |

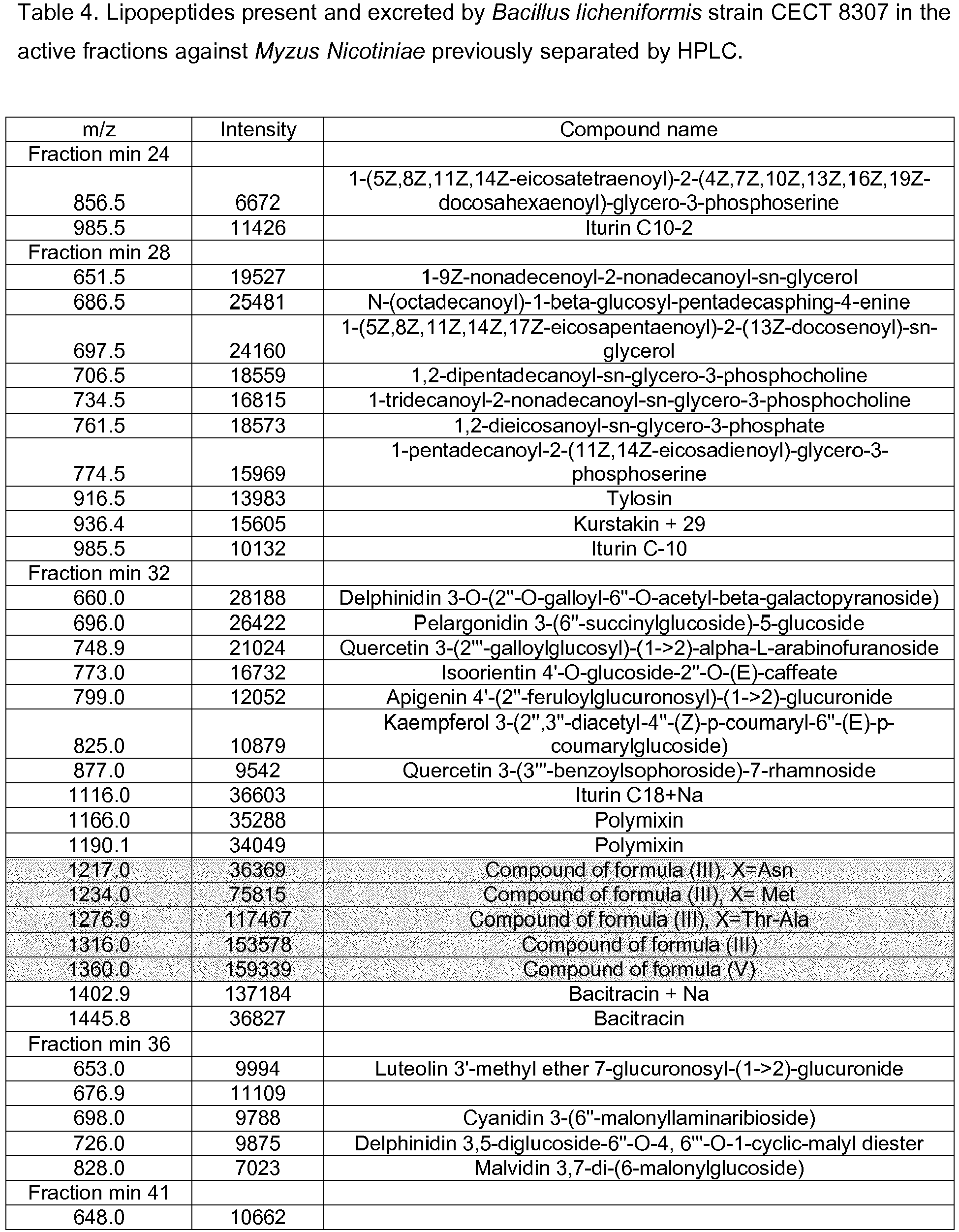

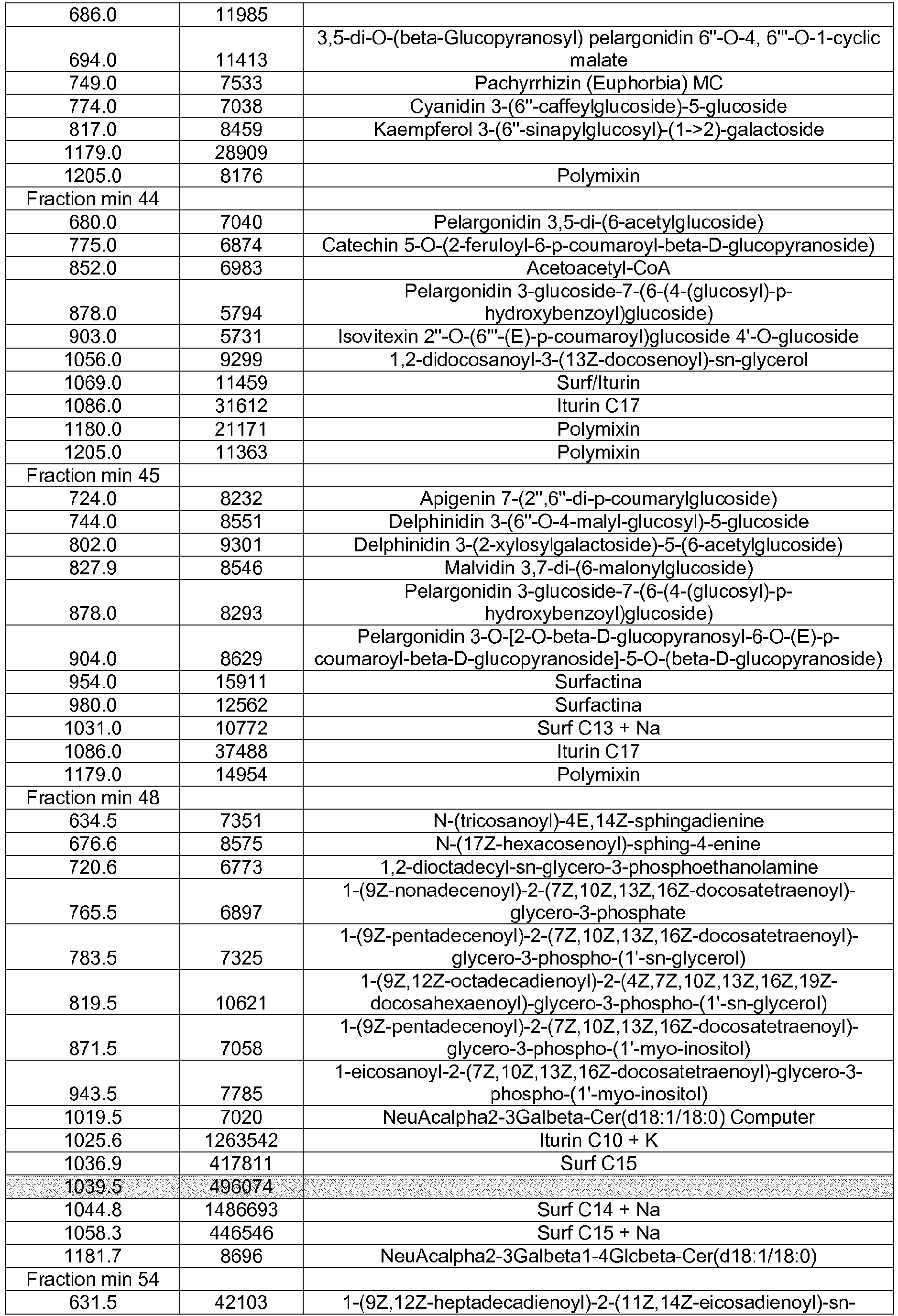

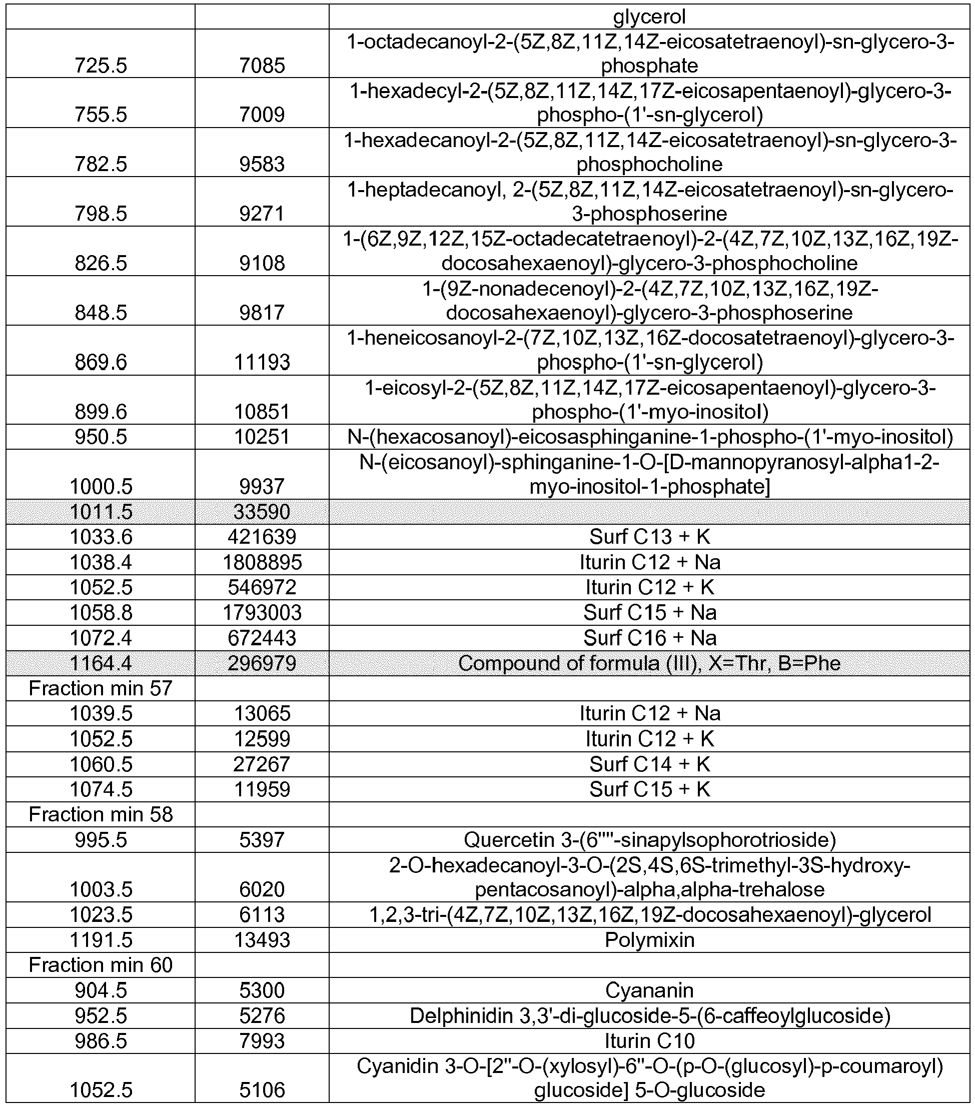

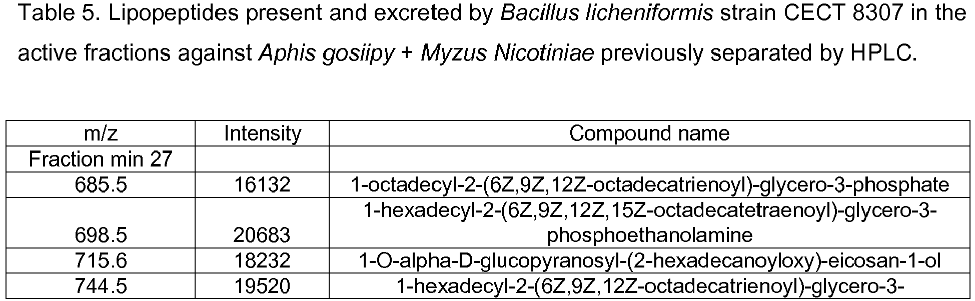

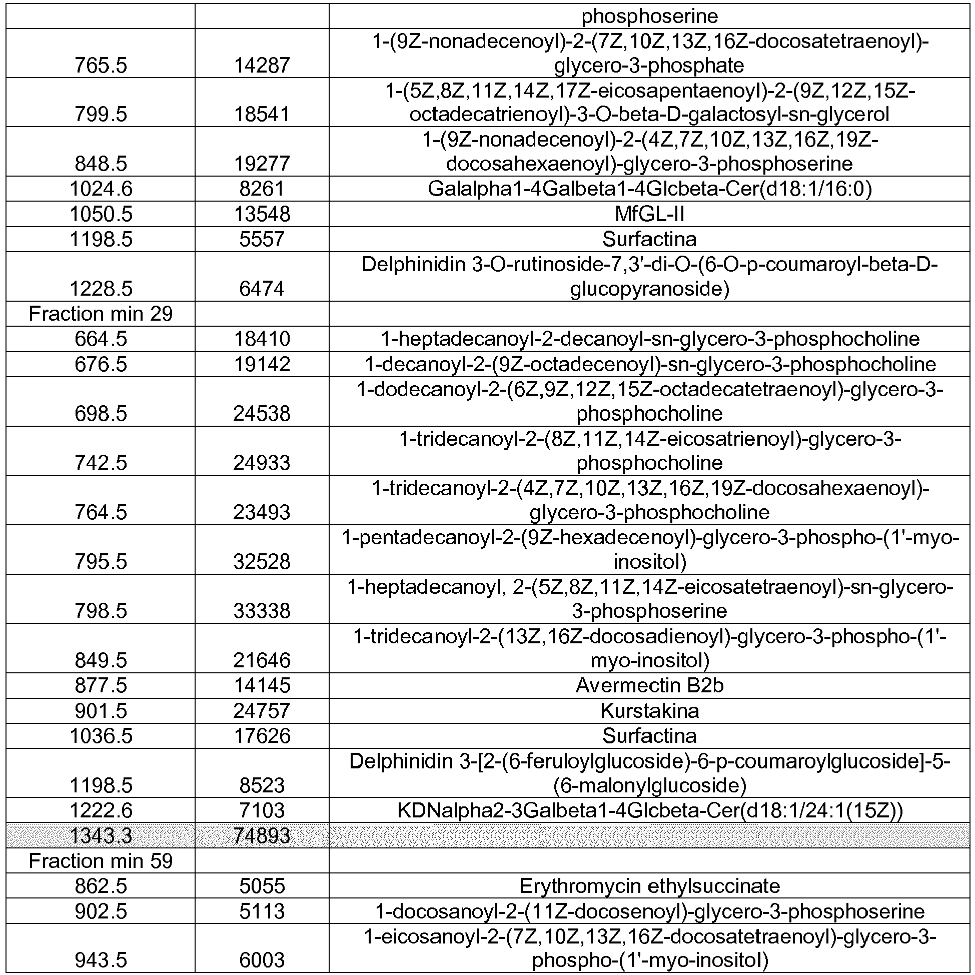

1. A *Bacillus licheniformis* strain deposited under deposit number CECT 8307, comprising the sequence identified by SEQ ID NO: 1.
2. A method for protecting or treating plants, plant parts, plant foliage, plant roots, plant seeds or fruits from insect infestations, comprising applying
   - a composition comprising *Bacillus licheniformis* strain deposited under deposit number CECT 8307, comprising the sequence identified by SEQ ID NO: 1 or
   - a supernatant obtained from a culture of *Bacillus licheniformis* strain deposited under deposit number CECT 8307, comprising the sequence identified by SEQ ID NO: 1 to said plant parts, plant foliage, plant roots, plant seeds or fruits.
3. Method according to claim 2, characterised in that said insect infestations are caused by foliar insects which attack the plant foliage or fruits.
4. Method according to any one of claims 2 or 3, characterised in that said insect infestations are caused by soil insects which attack plant roots.
5. Method according to any one of claims 2 to 4, characterised in that said insect infestations are caused by an insect selected from the group consisting of aphids, whiteflies and thrips.
6. Method according to any one of claims 2 to 5, characterised in that said insect infestations are caused by a member of a family selected from the group consisting of Aphididae, Aleyrodidae and Thripidae.
7. Method according to any one of claims 2 to 6, characterised in that said insect infestations are caused by an insect selected from the group consisting of *Myzus* spp., *Aphis* spp., *Bemisia* spp., *Trialeurodes* spp., *Frankliniella* spp. and *Thrips* spp.
8. Method according to any one of claims 2 to 7, characterised in that a biological or chemical pesticide is further applied to said plant parts, plant foliage, plant roots, plant seeds or fruits.
9. Method according to any one of claims 2 to 8, characterized in that *Bacillus licheniformis* strain deposited under deposit number CECT 8307 is applied as a whole broth culture.
10. Method according to any one of claims 2 to 9, characterized in that *Bacillus licheniformis* strain deposited under deposit number CECT 8307 is applied to the soil surrounding the plant roots.
11. Method according to any one of claims 2 to 10, characterized in that *Bacillus licheniformis* strain deposited under deposit number CECT 8307 is applied as wettable powders, granules, aqueous suspensions, emulsifiable concentrates or microencapsulations.
12. A compound of formula (I) 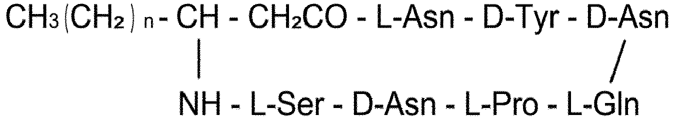
13. A compound of formula (II) 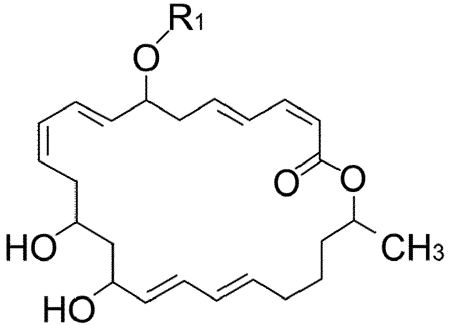 wherein R₁ is selected from the group consisting of H, CO-CH₂-COOH, CO-CH₂-CH₂-COOH and 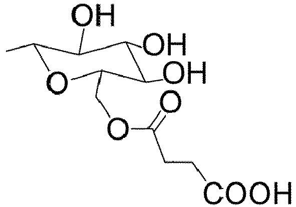
14. A compound of formula (III) 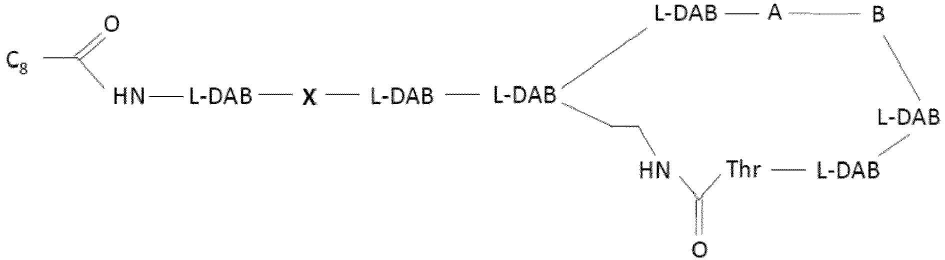 wherein X is selected from the group consisting of Met, Thr-Ala, Asn and Thr, A is selected from D-Leu and D-Phe, B is selected from the group consisting of Thr, Leu, Phe and ILeu, C₈ is 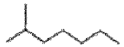 or 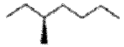 , L-DAB is 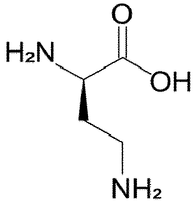 , provided that if X is Thr, B is Phe.
15. A compound of formula (IV) 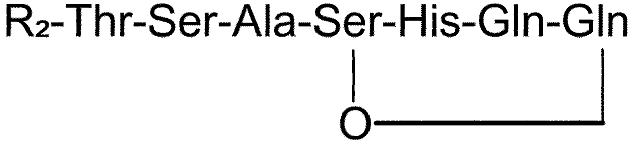 wherein R₂ is CH₃-CH₂-CH₂-CH₂-CH₂-CH₂-CH₂-CH₂-CH₂-CH₂-CH₂-.
16. A compound of formula (V) 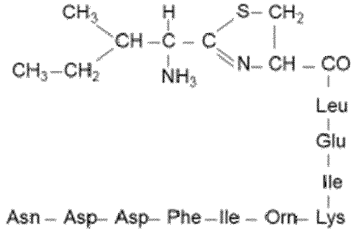
17. A method for protecting or treating plants, plant parts, plant foliage, plant roots, plant seeds or fruits from insect infestations, comprising applying a compound according to any one of claims 12-16 to said plant parts, plant foliage, plant roots, plant seeds or fruits.

## Claims

1. A *Bacillus licheniformis* strain deposited under deposit number CECT 8307, comprising the sequence identified by SEQ ID NO: 1.

2. A method for protecting or treating plants, plant parts, plant foliage, plant roots, plant seeds or fruits from insect infestations, comprising applying
- a composition comprising *Bacillus licheniformis* strain deposited under deposit number CECT 8307, comprising the sequence identified by SEQ ID NO: 1 or
- a supernatant obtained from a culture of *Bacillus licheniformis* strain deposited under deposit number CECT 8307, comprising the sequence identified by SEQ ID NO: 1 to said plant parts, plant foliage, plant roots, plant seeds or fruits.

3. Method according to claim 2, **characterised in that** said insect infestations are caused by foliar insects which attack the plant foliage or fruits.

4. Method according to any one of claims 2 or 3, **characterised in that** said insect infestations are caused by soil insects which attack plant roots.

5. Method according to any one of claims 2 to 4, **characterised in that** said insect infestations are caused by an insect selected from the group consisting of aphids, whiteflies and thrips.

6. Method according to any one of claims 2 to 5, **characterised in that** said insect infestations are caused by a member of a family selected from the group consisting of Aphididae, Aleyrodidae and Thripidae.

7. Method according to any one of claims 2 to 6, **characterised in that** said insect infestations are caused by an insect selected from the group consisting of *Myzus* spp., *Aphis* spp., *Bemisia* spp., *Trialeurodes* spp., *Frankliniella* spp. and *Thrips* spp.

8. Method according to any one of claims 2 to 7, **characterised in that** a biological or chemical pesticide is further applied to said plant parts, plant foliage, plant roots, plant seeds or fruits.

9. Method according to any one of claims 2 to 8, **characterized in that** *Bacillus licheniformis* strain deposited under deposit number CECT 8307 is applied as a whole broth culture.

10. Method according to any one of claims 2 to 9, **characterized in that** *Bacillus licheniformis* strain deposited under deposit number CECT 8307 is applied to the soil surrounding the plant roots.

11. Method according to any one of claims 2 to 10, **characterized in that** *Bacillus licheniformis* strain deposited under deposit number CECT 8307 is applied as wettable powders, granules, aqueous suspensions, emulsifiable concentrates or microencapsulations.

12. A compound of formula (I)

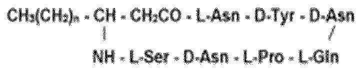

13. A compound of formula (II) 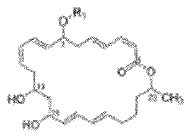 wherein R₁ is selected from the group consisting of H, CO-CH₂-COOH, CO-CH₂-CH₂-COOH and

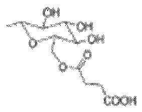

14. A compound of formula (III) 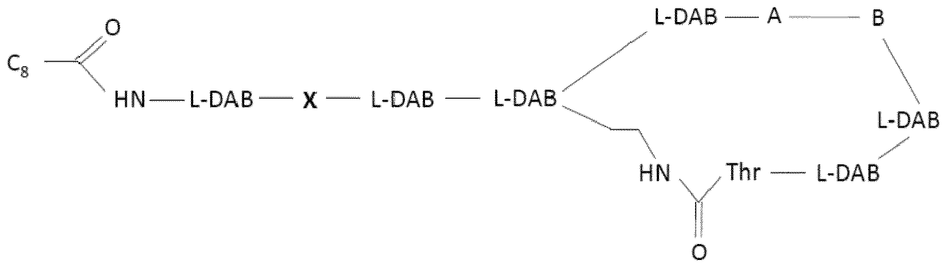 wherein X is selected from the group consisting of Met, Thr-Ala, Asn and Thr, A is selected from D-Leu and D-Phe, B is selected from the group consisting of Thr, Leu, Phe and ILeu, C₈ is 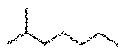 or 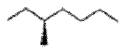 , L-DAB is 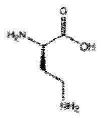 , provided that if X is Thr, B is Phe.

15. A compound of formula (IV) 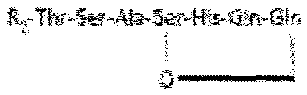 wherein R₂ is CH₃-CH₂-CH₂-CH₂-CH₂-CH₂-CH₂-CH₂-CH₂-CH₂-CH₂-.

16. A compound of formula (V)

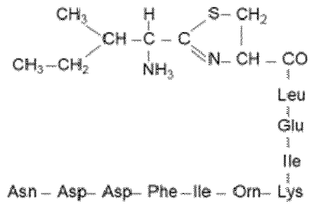

17. A method for protecting or treating plants, plant parts, plant foliage, plant roots, plant seeds or fruits from insect infestations, comprising applying a compound according to any one of claims 12-16 to said plant parts, plant foliage, plant roots, plant seeds or fruits.
